# EUROPEAN PATENT APPLICATION

(11) **EP 4 230 169 A1**
(43) Date of publication of application: **23.08.2023**
(21) Application number: 21879156.4
(22) Date of filing: 07.09.2021
(51) Int. Cl.: A61B 34/20, A61B 34/10, A61B 6/04, A61B 6/03, A61B 6/00

(54) **MEDICAL SYSTEM**

(30) Priority: 16.10.2020 CN 202011112976
(71) Applicant: Changzhou Lunghealth Medtech Company Limited, Jiangsu 213145 (CN)
(72) Inventor: LIU, Hongyi, Hangzhou, Jiangsu 213145 (CN)
(74) Representative: Renaudo, Adrien Hanouar
(86) International application number: PCT/CN2021/116891
(87) International publication number: WO 2022/078110

(57) **Abstract**

A medical system, comprises an operation table (10), a magnetic navigation apparatus (20), a medical image apparatus (30), and an optical positioning apparatus (40). The magnetic navigation apparatus (20) is configured to navigate, according to a medical path, a positioning medical tool (50) to a lesion region on the medical path. The optical positioning apparatus (40) comprises an imaging module (410) and at least three first markers (420) provided on the medical image apparatus (30). The optical positioning apparatus (40) is configured to acquire, according to the imaging of the at least three first markers (420) by the imaging module (410), the position of the medical image apparatus (30) relative to the imaging module (410). The medical image apparatus (30) is configured to move according to the position of the medical image apparatus (30) relative to the imaging module (410), so as to scan a head end region of the positioning medical tool (50) to acquire an image, and reconstruct a two-dimensional image and/or a three-dimensional model of the head end region of the positioning medical tool (50), so as to confirm the surrounding condition of the positioning medical tool (50) and whether the positioning medical tool reaches a correct position.

## Description

### CROSS-REFERENCE TO RELATED APPLICATIONS

The present disclosure refers to Chinese Patent Application No. 202011112976.X, filed on October 16, 2020, and entitled "Medical System", which is hereby incorporated by reference in its entirety.

### TECHNICAL FIELD

The present disclosure relates to medical technology field, and in particular, to a medical system.

### BACKGROUND

Diagnosis and treatment of pulmonary nodules have been increasingly important for early detection and treatment of lung cancer. More advanced diagnosis and treatment methods are that a magnetic navigation device plans a medical path from a natural airway to a lesion position through a medical image, navigates a medical tool to the lesion position along this medical path, and then performs corresponding treatment on the lesion.

However, the above lesion position is only the lesion position on the medical path planned by the magnetic navigation device through the previous medical image. Accompanied by respiratory movement and other actions of a patient and due to the possible deformation of an organ being a soft tissue, a relatively complicated registration process is required. The actual shape and position of trachea are not necessarily completely matched with the medical path, and an actual lesion position may deviate from a planned target position. And in the process of navigating the medical tool, only the previous medical image is used for auxiliary judgment, which may also result in poor accuracy and effective navigation efficiency of magnetic navigation.

### SUMMARY

In view of this, embodiments of the present disclosure provide a medical system for improving the accuracy and efficiency of conveying a medical tool to a lesion position.

The embodiments of the present disclosure provide a medical system, including an operation table, a magnetic navigation apparatus, a medical image apparatus, and an optical positioning apparatus.

The magnetic navigation apparatus is configured to navigate, according to a medical path, a positioning medical tool to a lesion region on the medical path.

The optical positioning apparatus includes an imaging module and at least three first markers provided on the medical image apparatus. The optical positioning apparatus is configured to acquire, according to the imaging of the at least three first markers by the imaging module, the position of the medical image apparatus relative to the imaging module.

The medical image apparatus is configured to move according to the position of the medical image apparatus relative to the imaging module, so as to scan a head end region of the positioning medical tool to acquire an image, and reconstruct a two-dimensional image and/or a three-dimensional model of the head end region of the positioning medical tool, so as to confirm the surrounding condition of the positioning medical tool.

Further, the imaging module is fixedly connected to the operation table and the position of the imaging module relative to the operation table is known.

Further, the optical positioning apparatus further includes at least three second markers provided on the operation table, and the optical positioning apparatus is configured to acquire, according to the imaging of the at least three second markers by the imaging module, the position of the imaging module relative to the operation table.

Further, the medical image apparatus includes an X-ray machine for imaging the head end region of the positioning medical tool to acquire a two-dimensional image.

Further, the medical image apparatus includes a CT machine for scanning the head end region of the positioning medical tool, acquiring CT data of a scanned region and reconstructing a two-dimensional image and a three-dimensional model of the head end region of the positioning medical tool.

Further, the imaging module includes at least two cameras with relative positions determined. A photographing range of the cameras covers the at least three first markers.

Further, the at least two cameras are infrared cameras, respectively.

Further, the magnetic navigation apparatus includes a magnetic field generator provided in the operation table and a positioning sensor provided in the positioning medical tool.

The magnetic field generator is configured to generate a positioning magnetic field to position the positioning sensor.

Further, the medical image apparatus is also configured to scan around a position of the positioning sensor acquired by the magnetic field generator to acquire a medical image, and to reconstruct a two-dimensional image and/or a three-dimensional model around the position, so as to confirm that the positioning medical tool has reached an actual lesion region.

Further, the acquired medical image includes an image of the position of the positioning sensor and an image of the lesion region. The medical image apparatus is configured to adjust and optimize own structures, parameters and characteristics according to the image of the position of the positioning sensor and an imaging range of the image of the lesion region as well as the characteristics of the lesion region, so as to ensure clear imaging of the lesion region.

Further, the medical system also includes a display module for performing a fused display on the two-dimensional image and/or three-dimensional model reconstructed by the medical image apparatus around the position and a two-dimensional image and/or three-dimensional model used for planning the medical path, so as to calibrate positioning of the positioning sensor and the medical path.

Further, the magnetic field generator is a flat plate-type generator that is movable in the operation table.

Further, the magnetic field generator is a hollow frame-type structure generator. A hollow structure of the frame-type structure generator allows to be transmitted by rays of the medical image apparatus.

Further, an upright is provided below the operation table. The upright is of a liftable structure.

Further, the operation table is movable relative to the upright in a plane of a table top.

Further, the medical image apparatus includes an X-ray tube for emitting X-rays and an imaging module for receiving the X-rays. The X-ray tube and the imaging module are separated on both sides of the operation table.

Further, the medical image apparatus also includes a C-shaped arm moving relative to the operation table. Both ends of the C-shaped arm are respectively configured to arrange the X-ray tube and the imaging module.

Further, the at least three first markers are respectively provided at different positions of the C-shaped arm.

In the embodiments of the present disclosure, an imaging module of an optical positioning apparatus acquires, by imaging at least three first markers provided on a medical image apparatus, the position of the medical image apparatus relative to the imaging module. A magnetic navigation apparatus acquires the position of a head end of a positioning medical tool and navigates, according to a medical path, the positioning medical tool to a lesion region targeted by the medical path. During the navigation, the medical image apparatus moves according to the position of the medical image apparatus relative to the imaging module, so as to scan a head end region of the positioning medical tool to acquire an image, and reconstruct a two-dimensional image and/or a three-dimensional model of the head end region of the positioning medical tool, so as to confirm the surrounding condition of the positioning medical tool, thus confirming whether the positioning medical tool correctly advances along a navigation path and whether the positioning medical tool has reached an actual lesion region. If an actual position of the positioning medical tool deviates from the medical path, the calibration compensation for positioning is performed through the medical image, so as to improve the accuracy of magnetic navigation. Meanwhile, the two-dimensional image and/or three-dimensional model of the head end region of the positioning medical tool reconstructed during the navigation may perform auxiliary judgment for a navigation action in real time, so as to ensure that the positioning medical tool is on an optimal path to reach the actual lesion region, whereby the positioning medical tool may quickly reach the actual lesion region, thereby improving the effective navigation efficiency of magnetic navigation.

### BRIEF DESCRIPTION OF THE DRAWINGS

The accompanying drawings described herein are used to provide a further understanding of the present disclosure, and constitute a part of the present disclosure. Exemplary embodiments of the present disclosure and the description thereof are used to explain the present disclosure, but do not constitute improper limitations to the present disclosure. In the drawings:
FIG. 1 is a schematic stereostructure diagram of a medical system according to an embodiment of the present disclosure;
FIG. 2 is another schematic stereostructure diagram of a medical system according to an embodiment of the present disclosure;
FIG. 3 is yet another schematic stereostructure diagram of a medical system according to an embodiment of the present disclosure;
FIG. 4a is a schematic stereostructure diagram of an operation table of a medical system according to an embodiment of the present disclosure;
FIG. 4b is another schematic stereostructure diagram of an operation table of a medical system according to an embodiment of the present disclosure;
FIG. 5 is yet another schematic stereostructure diagram of an operation table of a medical system according to an embodiment of the present disclosure;
FIG. 6 is a structural block diagram of a medical image apparatus of a medical system according to an embodiment of the present disclosure; and
FIG. 7 is a schematic stereostructure diagram of a medical image apparatus of a medical system according to an embodiment of the present disclosure.

### DETAILED DESCRIPTION OF EXAMPLE EMBODIMENTS

In order to make the objects, technical solutions and advantages of the present disclosure clearer, the technical solutions of the present disclosure will be clearly and completely described below in conjunction with specific embodiments of the present disclosure and the corresponding drawings. It is obvious that the described embodiments are only a part of the embodiments of the present disclosure, rather than all the embodiments. All other embodiments obtained by those ordinarily skilled in the art based on the embodiments in the present disclosure without involving creative efforts fall within the scope of protection of the present disclosure.

### Specific Embodiment

Embodiments of the present disclosure provide a medical system that combines a positioning sensor-based path navigation with a medical tool on the basis of a three-dimensional model of a bronchial tree and a three-dimensional model of blood vessels in the lung reconstructed from CT data, and provides an accessible passage for some special lesion positions, such as those peripheral to the lung that cannot be reached by a bronchoscope or those that do not have significant natural airway access, whereby the medical tool may reach these lesion positions to provide conditions for the treatment of these lesion positions.

In the following embodiments of the present disclosure, a positioning sensor, such as a positioning sensor provided on a positioning catheter or a positioning sensor provided on a biopsy tool, may be involved. First, the positioning sensors involved in the various embodiments of the present disclosure will be described.

These positioning sensors adopt the principle of electromagnetic navigation, unlike the general principle of magnetic navigation. The principle of magnetic navigation is mainly as follows: an external magnetic field is relied upon to attract or repel permanent magnets in an ablation catheter to influence the direction of medical instruments entering the body. The working principle of the positioning sensor in the embodiments of the present disclosure is mainly as follows: current is output to an external control system in response to a magnetic field in a space where the positioning sensor is located for a control system to position the corresponding catheter. In detail, the positioning sensor is in (signal) connection with the control system in a wired or wireless manner. The control system includes a magnetic field generator for generating a magnetic field in a certain range of positioning space. The positioning sensor is not magnetic per se, and a coil in the positioning sensor is configured to sense the magnetic field generated by the magnetic field generator. The magnetic field generator generates a varying magnetic field in a certain range of positioning space, and ensures that the magnetic field characteristic of each point in the positioning space is unique. The coil in the positioning sensor generates current in the varying magnetic field, the current is collected to generate a signal, the signal is then transmitted to the control system, and the control system analyzes the signal to determine an accurate position and direction of the corresponding catheter.

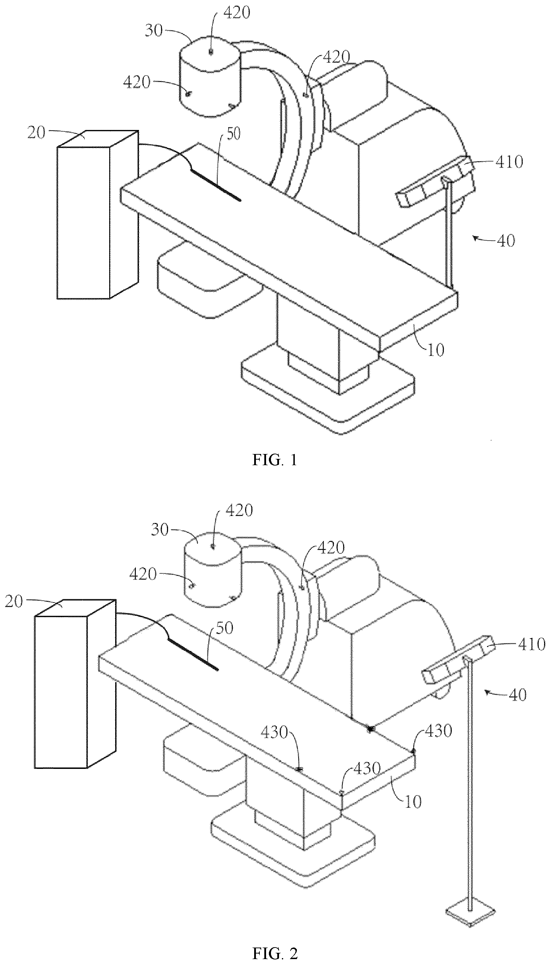

FIG. 1 is a schematic stereostructure diagram of a medical system according to an embodiment of the present disclosure. The medical system includes an operation table 10, a magnetic navigation apparatus 20, a medical image apparatus 30, and an optical positioning apparatus 40.

The magnetic navigation apparatus 20 is configured to navigate, according to a medical path, a positioning medical tool 50 to a lesion region on the medical path. The optical positioning apparatus 40 includes an imaging module 410 and at least three first markers 420 provided on the medical image apparatus 30. The optical positioning apparatus 40 is configured to acquire, according to the imaging of the at least three first markers 420 by the imaging module 410, the position of the medical image apparatus 30 relative to the imaging module 410. The medical image apparatus 30 is configured to move according to the position of the medical image apparatus 30 relative to the imaging module 410, so as to scan a head end region of the positioning medical tool 50 to acquire an image, and reconstruct a two-dimensional image and/or a three-dimensional model of the head end region of the positioning medical tool 50, so as to confirm the surrounding condition of the positioning medical tool 50.

The magnetic navigation apparatus 20 and the medical image apparatus 30 are respectively provided beside the operation table 10, and the operation table 10 can be transmitted by rays of the medical image apparatus 30. The optical positioning device 40 is not particularly limited herein, and it is only necessary to ensure that the working range thereof may cover the operation table 10 and the medical image apparatus 30.

The operation table 10 described herein includes, but is not limited to, an operating table in an operating room, or a hospital bed for diagnosis and treatment of a patient, etc. The medical image apparatus 30 includes, but is not limited to, a CT machine, or an X-ray machine, etc., which may be broadly referred to as an imaging apparatus of all medical images when conditions permit, for imaging the partial or whole body of a patient. An imaging type thereof includes, but is not limited to, a two-dimensional image. The medical image apparatus 30 described herein may emit corresponding medical rays, including, but not limited to, X-rays. The operation table 10 described herein can be transmitted by rays of the medical image apparatus 30 and has a function of no influence on an imaging effect of the medical image apparatus 30.

The magnetic navigation apparatus 20 includes a path planning module for determining a lesion region target to be reached according to a three-dimensional model of a bronchial tree and a three-dimensional model of blood vessels in the lung reconstructed from medical image data, mainly referred to herein as CT data, and planning a navigation path from a main carina to a target point via a natural airway, a navigation path from the main carina to a puncture starting point via the natural airway, and a puncture path from the puncture starting point to a puncture target point. These paths constitute a medical path reaching the lesion region via a natural orifice. In addition, the shape structure of the magnetic navigation apparatus 20 is not particularly limited in the embodiments of the present disclosure and in the present disclosure, and the shape structure and placement position of the magnetic navigation apparatus 20 in the drawings of the embodiments of the present disclosure are merely schematic.

Specifically, the magnetic navigation apparatus 20 first reconstructs a corresponding three-dimensional model according to medical image data, such as CT data, of the whole body or a lesion region of a patient acquired in advance. The lesion region described herein generally refers to an organ where a lesion exists. The three-dimensional model described herein is mainly used for planning a path and determining a lesion position, and may be referred to herein as a three-dimensional navigation model, so as to plan a medical path reaching the lesion region via a natural orifice. Then the positioning medical tool 50 is navigated to a lesion region on the medical path according to the medical path. The positioning medical tool 50 herein refers to a medical tool having a positioning function, such as a medical tool for biopsy, puncture and treatment, and may specifically be a guide wire, a catheter, a cell forceps, a biopsy needle, an inflatable balloon, an ablation catheter, etc.

The positioning medical tool 50 described herein is connected to the magnetic navigation apparatus 20, or part of the magnetic navigation apparatus 20. If the magnetic navigation apparatus 20 has a conveying mechanism similar to a mechanical arm, the conveying mechanism is connected to the positioning medical tool 50. The conveying mechanism drives the positioning medical tool 50 into the body of the patient according to the medical path and reaches a lesion region under the control of the magnetic navigation apparatus 20. This lesion region generally refers to a lesion region targeted by the medical path. The situation that the positioning medical tool 50 is conveyed into the body of the patient through the conveying mechanism has been described above. In other embodiments, it is also possible that the positioning medical tool 50 is conveyed into the body of the patient through a propulsion apparatus by means of the operation of a doctor, and the doctor manually controls the propulsion apparatus by means of a display device of the magnetic navigation apparatus 20, whereby the propulsion apparatus drives the positioning medical tool 50 to move in accordance with the medical path to reach the lesion region. This lesion region generally refers, certainly, to the lesion region targeted by the medical path.

The optical positioning apparatus 40 specifically includes at least three first markers 420 provided on the medical image apparatus 30 and the imaging module 410 for imaging the first markers 420. The imaging module 410 described herein includes, but is not limited to, a camera. The first markers 420 may be objects which can be photographed by the camera. The number of the first markers 420 is limited to at least three because the shape of the medical image apparatus 30 is fixed. The first markers 420 are provided in at least three different positions on the medical image apparatus 30, and these first markers 420 are imaged by the imaging module 410. The position of the medical image apparatus 30 is calibrated and identified using these first markers 420 as identification features, whereby the shape, position and imaging range of the C-shaped arm of the medical image apparatus 30 may be specified. During use, the position of the medical image apparatus 30 relative to the imaging module 410 may be acquired using an image analysis technology.

The imaging module 410 described herein specifically includes at least two cameras with relative positions determined. A photographing range of the cameras covers the at least three first markers 420. Specifically, the imaging module 410 described herein includes at least two cameras, i.e. at least binocular cameras, relative positions between the respective cameras are determined, and the respective cameras may photograph the at least three first markers 420, i.e. the photographing range of the respective cameras covers, at least, the at least three first markers 420. Herein, by combining the difference in pixels on images of the markers 420 photographed by the respective cameras and the difference in positions (directions and distances) between the respective cameras, the position of the imaging module 410 relative to the at least three first markers 420 may be calculated, i.e. the position of the medical image apparatus 30 relative to the imaging module 410 is acquired.

In addition, in other preferred embodiments of the present disclosure, in order to expand the application of the cameras so that the cameras are used in a case of poor light, to acquire high-quality images so as to improve the accuracy of an acquired relative position relationship, and also to avoid the interference of the cameras on the patient on the operation table 10 during photographing, at least two of the cameras are infrared cameras, respectively.

After obtaining the position of the medical image apparatus 30 relative to the imaging module 410, the medical image apparatus 30 may move according to the obtained relative position relationship. It is to be emphasized herein that the relative position between the imaging module 410 and the operation table 10 is known. That is, the above-mentioned position of the medical image apparatus 30 relative to the imaging module 410 may be converted into the position of the medical image apparatus 30 relative to the operation table 10. Specifically, the medical image apparatus 30 is moved to a photographing range to cover the head end region of the positioning medical tool 50. Then, the head end region of the positioning medical tool 50 is scanned to acquire a corresponding image. Then, a two-dimensional image and/or a three-dimensional model of the head end region of the positioning medical tool 50 are reconstructed. For example, corresponding CT data is acquired by irradiating and scanning the head end region of the positioning medical tool 50 through the CT machine, and then a two-dimensional image and a three-dimensional model of the head end region of the positioning medical tool 50 are reconstructed through these CT data; or a corresponding X-ray image is acquired by photographing the head end region of the positioning medical tool 50 through the X-ray machine, and then a two-dimensional image of the head end region of the positioning medical tool 50 is obtained by processing the X-ray image, or a three-dimensional model of the head end region of the positioning medical tool 50 is further reconstructed.

Herein, the purpose of reconstructing a two-dimensional image and/or a three-dimensional model of the head end region of the positioning medical tool 50 is to confirm the surrounding condition of the positioning medical tool 50, because the medical path where the navigation is performed by the magnetic navigation apparatus 20 is planned according to the previous medical image of the patient, and factors such as the respiratory action of the patient and organ deformation may affect an actual lesion position. That is, it is not necessarily accurate that the magnetic navigation apparatus 20 navigates the positioning medical tool 50 to the lesion position on the medical path according to the medical path. At this moment, it may be determined by means of the two-dimensional image and/or three-dimensional model whether the head end of the positioning medical tool 50 advances along a correct path and further whether the head end has reached an actual lesion region. If the head end has reached the nearby of the actual lesion region, the positioning medical tool 50 may be controlled to perform a corresponding treatment operation. If it is found that the head end of the positioning medical tool 50 does not reach the actual lesion region, a path reaching this position is re-planned according to a specific position of the head end of the positioning medical tool 50 and in combination with the lesion position on the two-dimensional image and/or the three-dimensional model. For example, the positioning, the path or the three-dimensional model is corrected and compensated, and the positioning medical tool 50 is controlled to move to an accurate lesion position according to the corrected position and navigation path, so as to perform further treatment operations.

It is to be noted herein that two algorithms are involved in the reconstruction of the two-dimensional image and/or the three-dimensional model obtained through the X-ray image. According to one of the algorithms, information such as the two-dimensional image, the medical path and the three-dimensional navigation model are fused and displayed in the same window so as to explicitly display the situation of the head end region of the positioning medical tool 50. According to the other algorithm, a two-dimensional X-ray image is further algorithmically reconstructed into a three-dimensional image of the head end region of the positioning medical tool 50 and the newly reconstructed three-dimensional model is used for correcting the positioning or navigation path.

It is conceivable that in the process that the magnetic navigation apparatus 20 navigates the positioning medical tool 50 according to the medical path, the medical image apparatus 30 may scan or photograph the head end region of the positioning medical tool 50 in real time according to a relative position to the optical positioning apparatus 40 or as needed so as to acquire a corresponding real-time medical image. In order to reduce the radiation to the patient, scanning or photographing is generally performed only as needed, and in order to ensure the quality of scanning or photographing, it is necessary to keep the positioning medical tool 50 stationary at this moment and to reconstruct a two-dimensional image and/or a three-dimensional model of the head end region of the positioning medical tool 50 so as to confirm whether the medical path deviates from an actual position of the lesion. Herein, determining whether the medical path deviates from an actual position of the lesion means determining whether the head end of the medical tool 50 deviates from a natural orifice (bronchus) through which the medical path is required to pass or the actual position of the lesion. If yes, the deviation may be corrected in time. Herein, deviation compensation is generally performed on the position of the head end of the medical tool based on the real-time medical image and the reconstructed model, or the registration is corrected, or the shape of the medical path/bronchial tree and the position of a target point are modified. If the deviation occurs, the deviation may be corrected in time so that the positioning medical tool 50 always remains on an actual path reaching an actual lesion position, whereby the positioning medical tool 50 may quickly and accurately reach the actual lesion position so as to perform further treatment operations to complete the diagnosis and treatment of the lesion, thereby improving the accuracy and efficiency of lesion treatment, shortening time for a treatment tool to intervene in the body of the patient, and alleviating discomfort or pain during diagnosis and treatment of the patient.

In addition, it is to be noted that the medical image apparatus 30 may reconstruct a two-dimensional image or a three-dimensional model of the head end region of the positioning medical tool 50 according to actual requirements. However, in order to ensure the judgment correctness of a movement path of the positioning medical tool 50 after entering the body of the patient, in a preferred embodiment, the medical image apparatus 30 may simultaneously reconstruct a two-dimensional image and a three-dimensional model of the head end region of the positioning medical tool 50, which are matched with the aforementioned three-dimensional navigation model reconstructed by CT for fused display, and the positioning or medical path is calibrated.

Further, in one implementation of the present disclosure, the imaging module 410 is fixedly connected to the operation table 10 and the position of the imaging module 410 relative to the operation table 10 is known.

Specifically, the imaging module 410 is fixedly connected to the operation table 10 through a connecting rod, and a relative position therebetween is known. That is, the position, direction and distance of the imaging module 410 relative to the operation table 10 are known. The first markers 420 on the medical image apparatus 30 are imaged and photographed by the imaging module 410 to acquire a relative position relationship of the medical image apparatus 30 relative to the imaging module 410. Since the relative position between the imaging module 410 and the operation table 10 is known, the relationship may be converted into a position relationship of the medical image apparatus 30 relative to the operation table 10, i.e. a position relationship of the medical image apparatus 30 and an imaging range thereof relative to the operation table 10 and the patient on the operation table 10, and then the medical image apparatus 30 may be guided to move so as to scan the head end region of the positioning medical tool 50 to acquire a corresponding image.

In addition, referring to FIG. 2 that is another schematic stereostructure diagram of a medical system according to an embodiment of the present disclosure, the optical positioning apparatus 40 further includes at least three second markers 430 provided on the operation table 10. The optical positioning apparatus 40 is configured to acquire, according to the imaging of the at least three second markers 430 by the imaging module 410, the position of the imaging module 410 relative to the operation table 10. The imaging module 410 described herein may be provided at any position where the operation table 10 and the medical image apparatus 30 can be photographed. Specifically, the imaging module is provided at any position where the first markers 420 and the second markers 430 can be photographed. As shown in the figure, the imaging module 410 is provided on a support standing beside the operation table 10 and separated from the operation table 10. The operation table 10 is provided with at least three of the second markers 430. The shape of the operation table 10 is determined as the medical image apparatus 30 described above, the specific shape thereof may be obtained by photographing at least three of the second markers 430 thereon, and the relative position relationship of the imaging module 410 relative to the operation table 10 may be acquired by using an image analysis technology.

In combination with the position relationship of the imaging module 410 relative to the medical image apparatus 30 acquired above, the position relationship between the medical image apparatus 30 relative to the operation table 10, i.e. the position relationship of the medical image apparatus 30 relative to the patient on the operation table 10 may be converted, and then the medical image apparatus 30 may be guided to move so as to scan the head end region of the positioning medical tool 50 to acquire a corresponding image.

Further, the medical image apparatus 30 includes an X-ray machine for imaging the head end region of the positioning medical tool 50 to acquire a two-dimensional image.

Specifically, the medical image apparatus 30 is an X-ray machine provided beside the operation table 10. The X-ray machine is capable of generating rays through a certain part of the body of the patient on the operation table 10. Herein, the rays mainly pass through a region around the head end of the positioning medical tool 50, and are then imaged. The imaging described herein includes, but is not limited to, digital imaging. And a corresponding two-dimensional image of this region is acquired. Further, the two-dimensional image may be algorithmically matched to the three-dimensional navigation model or algorithmically reconstructed into a three-dimensional model of the imaged region. The condition of the head end region of the positioning medical tool 50 described herein may be displayed on the two-dimensional image (and/or the reconstructed three-dimensional model), such as a relative position relationship with the medical tool, body tissues and bones, or a relative position relationship with the actual lesion region. The relative position relationship described herein includes distance and direction.

The condition of the head end of the positioning medical tool 50 may be mastered through the image of the head end region of the positioning medical tool 50 acquired above, and it is accurately determined whether the positioning medical tool 50 deviates from a path reaching the actual lesion position. Alternatively, it is confirmed whether the head end of the positioning medical tool 50 has reached the actual lesion region and that the direction of the head end of the positioning medical tool 50 has been aligned with the actual lesion region.

In addition, the medical image apparatus 30 also includes a CT machine for scanning the head end region of the positioning medical tool 50, acquiring CT data of a scanned region and reconstructing a two-dimensional image and a three-dimensional model of the head end region of the positioning medical tool 50.

Specifically, as mentioned above the medical image apparatus 30 is an X-ray machine. In other preferred embodiments of the present disclosure, the medical image apparatus 30 may also be a CT machine. The CT machine described herein is a computer X-ray tomography camera, which may scan a certain portion of the body of the patient on the operation table at a level with a certain thickness by means of rays, and may specifically scan a certain region of the body around the head end of the positioning medical tool 50 to generate corresponding medical images and then reconstruct a three-dimensional model of the head end region of the positioning medical tool 50 according to these medical images. The three-dimensional model described herein may more clearly master the condition of the head end of the positioning medical tool 50 than a two-dimensional image, so as to more accurately determine whether the positioning medical tool 50 deviates from a path reaching the actual lesion position or to accurately confirm whether the head end of the positioning medical tool 50 has reached the actual lesion region and more clearly determine whether the direction of the head end of the positioning medical tool 50 has been aligned with the actual lesion region.

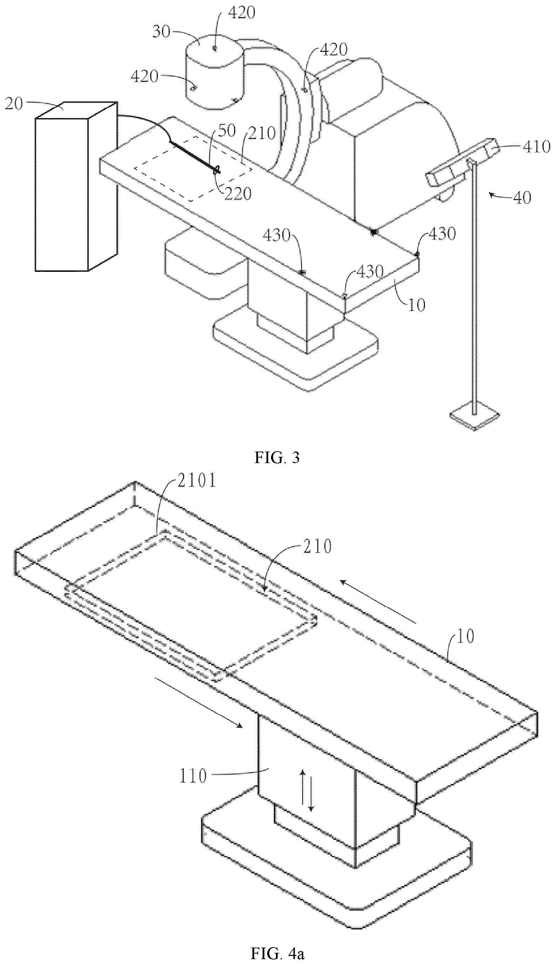

Further, referring to FIG. 3 that is another schematic stereostructure diagram of a medical system according to an embodiment of the present disclosure, the magnetic navigation apparatus 20 includes a magnetic field generator 210 provided in the operation table 10 and a positioning sensor 220 provided in the positioning medical tool 50. The magnetic field generator 210 is configured to generate a positioning magnetic field to position the positioning sensor 220.

Specifically, the magnetic field generator 210 is provided in the operation table 10, and the magnetic field generator 210 may emit a positioning magnetic field in the direction of a bed surface. When the patient lies on the operation table 10, the body of the lesion region thereof is located in the positioning magnetic field, and since the patient is generally in a general anesthesia state on the operation table 10, a relative position of the lesion region in the positioning magnetic field is fixed. Since the positioning magnetic field has its own coordinate system, referred to as a magnetic field coordinate system, a relative position of the lesion region in the magnetic field coordinate system is fixed, and the position and direction of the positioning medical tool 50 in the body of the patient may be represented by position coordinates of the head of the positioning medical tool 50 in the magnetic field coordinate system. Specifically, the positioning magnetic field may position the positioning sensor 220 in the positioning medical tool 50. Since the positioning sensor 220 is provided at the head end position of the positioning medical tool 50, corresponding position coordinates in the magnetic field coordinate system are acquired by positioning the positioning sensor 220, thus obtaining the position and direction of the positioning medical tool 50 in the body of the patient. Alternatively, the positioning sensor 220 is provided inside the positioning medical tool 50 and a relative position between the positioning sensor and the head thereof is known, position coordinates of the head end of the positioning medical tool 50 in the magnetic field coordinate system are acquired by conversion, and the position and direction of the positioning medical tool 50 in the body of the patient are further obtained.

Furthermore, the medical image apparatus 30 is also configured to scan around a position of the positioning sensor 220 acquired by the magnetic field generator 210 to acquire a medical image, and to reconstruct a two-dimensional image and/or a three-dimensional model around the position, so as to confirm the surrounding condition of the positioning medical tool 50 and whether the positioning medical tool has reached an actual lesion region.

Specifically, the positioning medical tool 50 with the positioning sensor 220 is operated by a doctor or conveyed into the body of the patient by the magnetic navigation apparatus 20, and the positioning medical tool 50 is navigated to the lesion region on the medical path according to the medical path. At this moment, the medical image apparatus 30 scans the position of the positioning sensor 220 in the magnetic field generator 210. That is, a certain range around the position of the positioning coordinates of the positioning sensor 220 in the magnetic field coordinate system is scanned to acquire a corresponding medical image, and a two-dimensional image and/or a three-dimensional model around the position are reconstructed according to the acquired medical image. The reconstructed two-dimensional image and/or three-dimensional model described herein include an image or a model of the head end part of the positioning medical tool 50, which may certainly determine the position and orientation of the head end of the positioning medical tool 50, and may also include an actual lesion of the patient and a natural orifice (bronchus) where the lesion is located. If the head end of the positioning medical tool in this image or model has entered the actual lesion of the patient, it may be confirmed that the positioning medical tool 50 has reached the actual lesion region. Meanwhile, a relative position relationship between the head end of the positioning medical tool 50 and the lesion region may be further acquired. The relative position relationship mainly refers to the distance and relative direction between the head end of the positioning medical tool 50 and the actual lesion region and the orientation of the head end of the positioning medical tool 50. If the head end of the positioning medical tool in this image or model has not yet entered the actual lesion of the patient, it is indicated that the positioning medical tool 50 has not reached the actual lesion region, and it may be possible that the actual lesion region is not on the medical path. At this moment, the medical path needs to be readjusted or the position needs to be corrected according to the two-dimensional image and/or three-dimensional model, whereby the magnetic navigation apparatus 20 navigates the positioning medical tool 50 to the actual lesion region. At this moment, the scanning range of the medical image apparatus 30 may need to be expanded, and a larger range of two-dimensional image and/or three-dimensional model is then acquired to ensure coverage of the actual lesion region.

It is to be noted herein that with reference to the above description, the reconstruction of the two-dimensional image and/or the three-dimensional model according to the medical image is determined by the type of the medical image acquired by the medical image apparatus 30 and a reconstruction algorithm, i.e. by a specific type of the medical image apparatus 30.

Further, the medical image acquired above includes an image of the position of the positioning sensor 220 and an image of the lesion region. The medical image apparatus 30 is configured to adjust and optimize own structures, parameters and characteristics according to the image of the position of the positioning sensor 220 and an imaging range of the image of the lesion region as well as the characteristics of the lesion region, so as to ensure clear imaging of the lesion region.

Specifically, the medical image apparatus 30 may cover a certain range of the medical image acquired by scanning around the position of the positioning sensor 220 acquired by the magnetic field generator 210. The range may include an image of the position of the positioning sensor 220 and an image of the lesion region, and the image of the lesion region at this moment may not be clear enough. Especially for a conventional X-ray device, since the device needs to universally adapt to different working conditions (such as orthopedics and heart), and for lesions with a relatively small density difference such as inflammation and nodules (close to the density of normal tissues), images that may be obtained can only show non-obvious shadows or cannot be distinguished. However, images acquired and reconstructed by small-scale CT may also fail to clearly distinguish the lesion (contour, position and size) with small difference from the density of surrounding normal tissues due to the structural limitations (influence on the imaging capability due to factors such as power and heat dissipation). The structure and imaging module design of the medical image apparatus 30 is optimized for lesions with small density differences. And after the positioning medical tool 50 is navigated to the lesion region on the medical path according to the medical path, the medical image acquired by the medical image apparatus 30 on the region around the position of the positioning sensor 220 may include a part of images of the positioning medical tool 50, i.e. an image of the position of the positioning sensor 220 and an image of the lesion region. At this moment, the medical image apparatus 30 may optimally adjust the parameters and characteristics when acquiring the medical image according to the imaging range of the image acquired above and the characteristics of the lesion, thus making it possible to improve the image quality in view of the problem that a conventional medical image device cannot clearly image lesions with small density differences from the surrounding tissues. Furthermore, the parameters and characteristics of the medical image apparatus 30 may be re-optimally adjusted according to the previous imaging result of the medical image and a second clear imaging may be performed, thereby distinguishing the contour, position and size of the lesion region.

In addition, it is to be noted that the medical image apparatus 30 may reconstruct a two-dimensional image or a three-dimensional model of the head end region of the positioning medical tool 50 according to actual requirements. However, in order to ensure the judgment correctness of a movement path of the positioning medical tool 50 after entering the body of the patient, in a preferred embodiment, the medical system further includes a display module (not shown in the figure). The medical image apparatus 30 may simultaneously reconstruct a two-dimensional image and/or a three-dimensional model of the head end region of the positioning medical tool 50, and the two-dimensional image and/or the three-dimensional model may be correspondingly used when planning the medical path, i.e. the aforementioned two-dimensional navigation image and/or three-dimensional model before navigation. The display module is configured to perform a fused display on the two-dimensional images and/or three-dimensional models acquired twice above, i.e. to match and align the positions and features of the two-dimensional images and/or three-dimensional models acquired twice above on the display module and to display in a view simultaneously for clear observation, so as to calibrate the positioning of the positioning sensor 220 and the medical path.

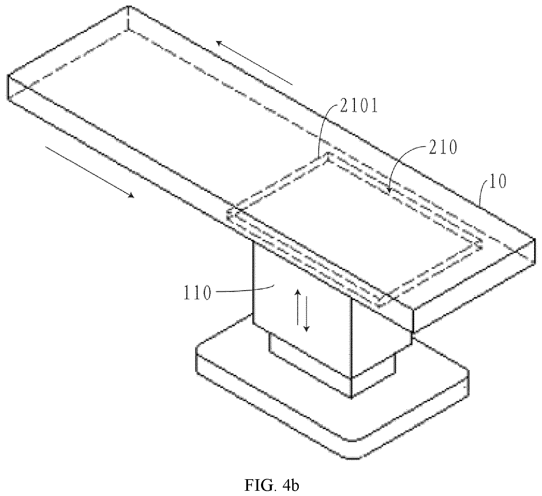

Referring to FIGs. 4a and 4b, in a feasible implementation of an embodiment of the present disclosure, the magnetic field generator 210 is a flat plate-type generator 2101 that is movable in the operation table 10.

Specifically, the flat plate-type generator 2101 is provided in the operation table 10 and is freely movable in the operation table 10. For example, a slide rail (not shown in the figure) is provided inside the operation table 10 along a length direction thereof. Two opposite sides of the flat plate-type generator 2101 are respectively provided on the slide rail so that the flat plate-type generator 2101 is freely movable in the operation table 10. In order to prevent the magnetic field emitted by the magnetic field generator 210 from interfering with the medical image apparatus 30 or prevent the magnetic field generator 210 from shielding the rays of the medical image apparatus 30 to reduce the image quality if the medical image apparatus 30 is to be used for scanning or photographing, the flat plate-type generator 2101 may be temporarily moved outside the scanning or photographing range of the medical image apparatus 30 to ensure that the medical image acquired by the medical image apparatus 30 is clear and accurate.

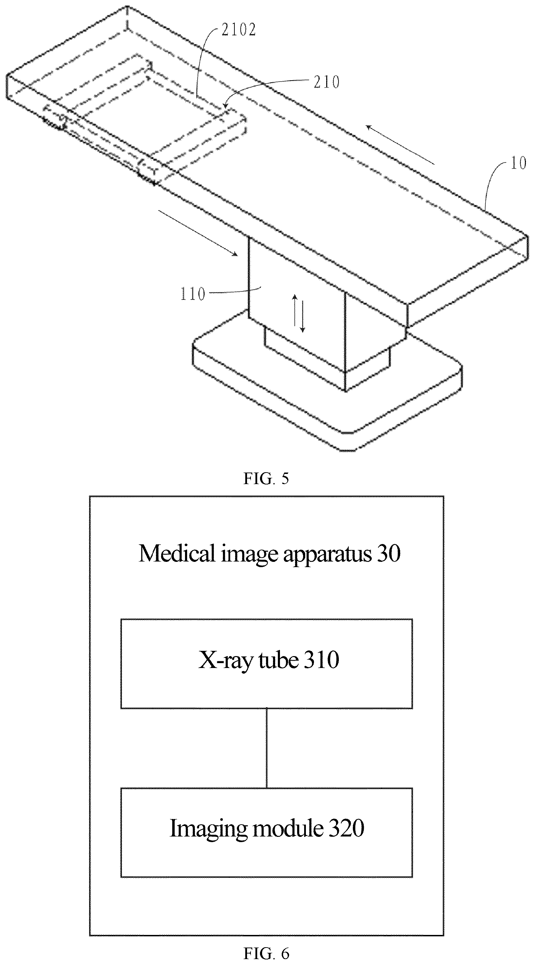

In addition, referring to FIG. 5, in another feasible implementation of an embodiment of the present disclosure, the magnetic field generator 210 is a hollow frame-type structure generator 2012. A hollow structure of the frame-type structure generator 2102 allows to be transmitted by rays of the medical image apparatus 30.

Specifically, the hollow frame-type structure generator 2012 includes two magnetic field generators provided oppositely and a connecting structure connecting two ends of the two magnetic field generators, which are connected end-to-end to form a hollow frame-type structure. Herein, the connecting structure is made of a ray-transmittable material of the medical image apparatus 30, including but not limited to carbon fiber or plastic, etc. Herein, the hollow structure may be passed through by the rays of the medical image apparatus 30, and does not affect the transmission of the rays of the medical image apparatus when the medical image apparatus 30 needs to scan or photograph the patient. Thus, while the magnetic navigation apparatus 20 operates, the medical image apparatus 30 may also acquire a corresponding medical image without interfering with each other, thereby improving the efficiency of the medical system.

Further, referring to FIGs. 4a, 4b and 5, in other preferred embodiments of the present disclosure, an upright 110 is provided below the operation table 10. The upright 110 is of a liftable structure.

Specifically, the upright 110 functions to support the operation table 10, and the upright 110 of the liftable structure includes, but is not limited to, a hydraulic lifting structure. By means of the upright 110, a table top of the operation table 10 may be moved in a vertical direction, such as an arrow direction shown in the figure, so as to adapt to a variety of different operation heights, thereby expanding the range of application of the medical system.

Furthermore, in other preferred embodiments of the present disclosure, the operation table 10 is movable relative to the upright 110 in a plane of a table top.

Specifically, a plane of the table top of the operation table 10 is movable at the top end of the upright 110. It is to be emphasized herein that the plane is movable back and forth along the length direction of the operation table 10, such as the arrow direction shown in the figure, or it is movable arbitrarily in the plane of the table top of the operation table 10. For example, the plane of the table top of the operation table 10 rotates relative to the top end of the upright 110. This is designed in consideration that a patient to be treated on the operation table 10 generally needs to be treated with general anesthesia. That is, there is no relative position between the operation table 10 and the patient. If a photographed or scanned part of the patient on the medical image apparatus 30 is changed, the position of the operation table 10 only needs to be moved at this moment to realize the change of the photographed or scanned part of the patient without waking up the patient, so as to improve the diagnosis and treatment efficiency of the medical system.

In addition, referring to FIG. 6 that is a structural block diagram of a medical image apparatus of a medical system according to an embodiment of the present disclosure, the medical image apparatus 30 includes an X-ray tube 310 for emitting X-rays and an imaging module 410 for receiving the X-rays. The X-ray tube 310 and the imaging module 410 are separated on both sides of the operation table 10. It is not limited whether the X-ray tube 310 or the imaging module 410 is provided above the operation table.

Herein, the X-ray tube 310 emits X-rays to cover an imaging range of the imaging module 410. The X-ray tube 310 and the imaging module 410 are arranged oppositely and respectively provided on both sides of the operation table 10, whereby the rays emitted from the X-ray tube 310 may pass through the body of the patient on the operation table 10, reach the imaging module 410, and be imaged on the imaging module 410, so as to obtain a medical image of a part of the patient on the operation table 10 through which the rays pass.

The medical image apparatus 30 further includes a reconstruction module and a display module. The reconstruction module is configured to reconstruct a two-dimensional image and/or a three-dimensional model according to the acquired medical image. The display module is configured to correspondingly display the two-dimensional image and/or the three-dimensional model.

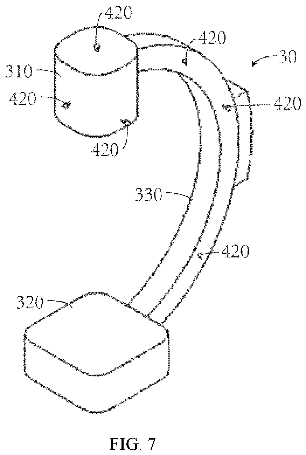

Further, referring to FIG. 7, in a feasible implementation of an embodiment of the present disclosure, the medical image apparatus 30 also includes a C-shaped arm 330 moving relative to the operation table 10. Both ends of the C-shaped arm 330 are respectively configured to arrange the X-ray tube 310 and the imaging module 410. Herein, two end portions of the C-shaped arm 330 are the X-ray tube 310 and the imaging module 410, respectively, and a C opening faces the operation table 10. Herein, the movement of the C-shaped arm 330 relative to the operation table 10 means that the opening of the C-shaped arm 330 is movable along the length or width direction of the operation table 10, and may also mean that the opening of the C-shaped arm 330 rotates around the operation table 10, whereby the photographing or scanning range of the medical image apparatus 30 covers the entire operation table 10 and various directions thereof. Medical images may be captured at appropriate angles as needed to enhance the use experience of the medical system.

In addition, in order that the imaging module 410 determines the shape of the medical image apparatus 30 more accurately so as to acquire the position of the medical image apparatus 30 relative to the imaging module 410 through the image analysis technology, the at least three first markers are generally respectively provided at different positions of the C-shaped arm 330. That is, by calibrating and identifying the C-shaped arm 330 using at least three different key points of the C-shaped arm 330 as identification features, a specific shape of the C-shaped arm 330 with a relatively fixed shape may be acquired. In addition, since the positions of other components (the X-ray tube 310 and the imaging module 410) of the medical image apparatus 30 on the C-shaped arm 330 are relatively determined, the shape, position and imaging range of the entire medical image apparatus 30 may be acquired, and then the relative position relationship between the medical image apparatus 30 and the imaging module 410 may be acquired by using the image analysis technology.

Further, in order to further verify the acquired shape of the medical image apparatus 30, the first markers 420 may be provided on the X-ray tube 310 and/or the imaging module 410. The first markers are imaged by the imaging module 410 to verify the shape acquired above.

Furthermore, it is mentioned above that at least three of the second markers 430 are provided on the operation table 10 and imaged by the imaging module 410, and the position relationship between the operation table 10 and the imaging module 410 is acquired by using the image analysis technology. Herein, the second markers 430 also need to be limited, i.e. at least three of the second markers 430 are respectively provided at different positions on the operation table 10. For the detailed reasons, reference may be made to the description of the positions at which the first markers 420 are provided in the above embodiments. The detailed descriptions thereof will be omitted herein.

Those skilled in the art will appreciate that the embodiments of the present disclosure may be provided as a method, a system, or a computer program product. Therefore, the present disclosure may adopt forms of complete hardware embodiments, complete software embodiments, or embodiments integrating software and hardware. Moreover, the present disclosure may adopt the form of a computer program product implemented on one or more computer-available storage media (including, but not limited to, a disk memory, a CD-ROM, an optical memory, etc.) containing computer-available program codes.

The present disclosure is described with reference to flowcharts and/or block diagrams of the method, the device (system), and the computer program product according to the embodiments of the present disclosure. It will be appreciated that each flow and/or block in the flowcharts and/or the block diagrams and a combination of the flows and/or the blocks in the flowcharts and/or the block diagrams may be implemented by computer program instructions. These computer program instructions may be provided for a general-purpose computer, a special-purpose computer, an embedded processor, or processors of other programmable data processing devices to generate a machine, whereby an apparatus for implementing functions specified in one or more flows of the flowcharts and/or one or more blocks of the block diagrams is generated via instructions executed by the computers or the processors of the other programmable data processing devices.

These computer program instructions may also be stored in a computer-readable memory capable of guiding the computers or the other programmable data processing devices to work in a specific manner, whereby a manufactured product including an instruction apparatus is generated via the instructions stored in the computer-readable memory. The instruction apparatus implements the functions specified in one or more flows of the flowcharts and/or one or more blocks of the block diagrams.

These computer program instructions may also be loaded to the computers or the other programmable data processing devices, whereby processing implemented by the computers is generated by performing a series of operation steps on the computers or the other programmable devices. Thus, the instructions executed on the computers or the other programmable devices provide a step of implementing the functions specified in one or more flows of the flowcharts and/or one or more blocks of the block diagrams.

In a typical configuration, a computing device includes one or more central processing units (CPUs), an input/output interface, a network interface, and a memory.

The memory may include a non-persistent memory, a random access memory (RAM) and/or a non-volatile memory, and other forms in a computer-readable medium, such as a read-only memory (ROM) or a flash RAM. The memory is an example of the computer-readable medium.

The computer-readable medium includes non-volatile and volatile, removable and non-removable media. Information may be stored in any way or by any technology. The information may be computer-readable instructions, data structures, modules of programs, or other data. Examples of the computer storage medium include, but are not limited to, a phase-change random access memory (PRAM), a static random access memory (SRAM), a dynamic random access memory (DRAM), other types of RAMs, a ROM, an electrically erasable programmable read-only memory (EEPROM), a flash memory or other memory technologies, a CD-ROM, a digital versatile disc (DVD) or other optical memories, a cassette tape, a tape and disk memory or other magnetic memories or any other non-transport media. The non-volatile storage medium may be used for storing computing device-accessible information. As defined herein, the computer-readable medium does not include computer-readable transitory media, such as modulated data signals and carrier waves.

It is also to be noted that the term "including", "comprising", or any other variations thereof are intended to cover a non-exclusive inclusion, whereby processes, methods, articles, or devices including a series of elements not only include those elements, but also include those elements that are not explicitly listed, or include elements inherent to such processes, methods, articles, or devices. The elements defined by a statement "include a..." do not exclude the condition that other same elements also exist in the processes, methods, articles, or devices including the elements under the condition that no more restraints are required.

Those skilled in the art will appreciate that the embodiments of the present disclosure may be provided as a method, a system, or a computer program product. Therefore, the present disclosure may adopt forms of complete hardware embodiments, complete software embodiments, or embodiments integrating software and hardware. Moreover, the present disclosure may adopt the form of a computer program product implemented on one or more computer-available storage media (including, but not limited to, a disk memory, a CD-ROM, an optical memory, etc.) containing computer-available program codes.

The above descriptions are merely the embodiments of the present disclosure and are not intended to limit the present disclosure. It will be apparent to those skilled in the art that various modifications and variations may be made in the present disclosure. Any modification, equivalent replacement, improvement, etc. made within the spirit and principle of the present disclosure may be included within the scope of the claims appended to the present disclosure.

## Claims

1. A medical system, comprising an operation table, a magnetic navigation apparatus, a medical image apparatus, and an optical positioning apparatus; wherein,
the magnetic navigation apparatus is configured to navigate, according to a medical path, a positioning medical tool to a lesion region on the medical path;
the optical positioning apparatus comprises an imaging module and at least three first markers provided on the medical image apparatus, and the optical positioning apparatus is configured to acquire, according to the imaging of the at least three first markers by the imaging module, position of the medical image apparatus relative to the imaging module; and
the medical image apparatus is configured to move according to the position of the medical image apparatus relative to the imaging module, so as to scan a head end region of the positioning medical tool to acquire an image, and reconstruct a two-dimensional image and/or a three-dimensional model of the head end region of the positioning medical tool, so as to confirm the surrounding condition of the positioning medical tool.

2. The system according to claim 1, wherein the imaging module is fixedly connected to the operation table and the position of the imaging module relative to the operation table is known.

3. The system according to claim 1, wherein the optical positioning apparatus further comprises at least three second markers provided on the operation table, and the optical positioning apparatus is configured to acquire, according to imaging of the at least three second markers by the imaging module, the position of the imaging module relative to the operation table.

4. The system according to claim 1, wherein the medical image apparatus comprises an X-ray machine for imaging the head end region of the positioning medical tool to acquire a two-dimensional image.

5. The system according to claim 1, wherein the medical image apparatus comprises a CT machine for scanning the head end region of the positioning medical tool, acquiring CT data of a scanned region and reconstructing a two-dimensional image and a three-dimensional model of the head end region of the positioning medical tool.

6. The system according to claim 1, wherein the imaging module comprises at least two cameras with relative positions determined, a photographing range of the cameras covering the at least three first markers.

7. The system according to claim 6, wherein the at least two cameras are infrared cameras, respectively.

8. The system according to claim 1, wherein the magnetic navigation apparatus comprises a magnetic field generator provided in the operation table and a positioning sensor provided in the positioning medical tool; and
the magnetic field generator being configured to generate a positioning magnetic field to position the positioning sensor.

9. The system according to claim 8, wherein the medical image apparatus is further configured to scan around a position of the positioning sensor acquired by the magnetic field generator to acquire a medical image, and to reconstruct a two-dimensional image and/or a three-dimensional model around the position, so as to confirm that the positioning medical tool has reached an actual lesion region.

10. The system according to claim 9, wherein the acquired medical image comprises an image of the position of the positioning sensor and an image of the lesion region, and the medical image apparatus is configured to adjust and optimize own structures, parameters and characteristics according to the image of the position of the positioning sensor and an imaging range of the image of the lesion region as well as the characteristics of the lesion region, so as to ensure clear imaging of the lesion region.

11. The system according to claim 9, further comprising: a display module for performing a fused display on the two-dimensional image and/or three-dimensional model reconstructed by the medical image apparatus around the position and a two-dimensional image and/or three-dimensional model used for planning the medical path, so as to calibrate positioning of the positioning sensor and the medical path.

12. The system according to any one of claims 8 to 11, wherein the magnetic field generator is a flat plate-type generator that is movable in the operation table.

13. The system according to any one of claims 8 to 11, wherein the magnetic field generator is a hollow frame-type structure generator, a hollow structure of the frame-type structure generator allowing to be transmitted by rays of the medical image apparatus.

14. The system according to claim 1, wherein an upright is provided below the operation table, the upright being of a liftable structure.

15. The system according to claim 14, wherein the operation table is movable relative to the upright in a plane of a table top.

16. The system according to any one of claims 1 to 9, wherein the medical image apparatus comprises an X-ray tube for emitting X-rays and an imaging module for receiving the X-rays, the X-ray tube and the imaging module being separated on both sides of the operation table.

17. The system according to claim 16, wherein the medical image apparatus further comprises a C-shaped arm moving relative to the operation table, both ends of the C-shaped arm being respectively configured to arrange the X-ray tube and the imaging module.

18. The system according to claim 17, wherein the at least three first markers are respectively provided at different positions of the C-shaped arm.
